# FASCICULE DE BREVET EUROPEEN

(11) **EP 0 968 171 B1**
(45) Date de publication et mention de la délivrance du brevet: **08.10.2003**
(21) Numéro de dépôt: 98938744.4
(22) Date de dépôt: 13.07.1998
(51) Int. Cl.: C07C 211/63, C07C 217/84, A61K 7/13, C07D 295/14

(54) **BASES D'OXYDATION CATIONIQUES, LEUR UTILISATION POUR LA TEINTURE D'OXYDATION DES FIBRES KERATINIQUES, COMPOSITIONS TINCTORIALES ET PROCEDES DE TEINTURE**
KATIONISCHE OXIDATIONSBASEN, DEREN VERWENDUNG ZUR OXYDATIONSFÄRBUNG VON KERATINISCHEN FASERN, FÄRBEZUSAMMENSETZUNGEN UND VERFAHREN
CATIONIC OXIDATION BASES, THEIR USE FOR OXIDATION DYEING OF KERATIN FIBRES, DYEING COMPOSITIONS AND DYEING METHODS

(30) Priorité: 16.07.1997 FR 9709027
(43) Date de publication de la demande: 05.01.2000
(60) Demande divisionnaire: 02290042.7
(73) Titulaire: L'OREAL, 75008 Paris (FR)
(72) Inventeur: GENET, Alain, F-93600 Aulnay sous Bois (FR); LAGRANGE, Alain, F-77770 Coupvray (FR)
(74) Mandataire: Goulard, Sophie
(86) Numéro de dépôt international: FR9801534
(87) Numéro de publication internationale: WO99003819

(56) Documents cités:
- EP-A- 0 673 926
- DE-B- 1 292 784
- US-A- 5 139 532

## Description

L'invention a pour objet des bases d'oxydation monobenzéniques comportant au moins un groupement cationique Z, Z étant choisi parmi des chaînes aliphatiques quaternisées et des chaînes aliphatiques contenant au moins un cycle saturé quaternisé, leur utilisation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre. de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

On connaît dans la demande EP 673 926 des composés 2-nitro paraphénylènediamines souffrés en position 5 qui sont éventuellement substitués sur le souffre par un ammonium quaternaire. Ils sont utilisés pour la coloration directe des cheveux. Le document DE 1 292 784 décrit également des compositions de teinture des cheveux comprenant des colorants irects du type aza obtenus par réaction de dérivés de paraphénylènediamines comportant un groupe nitro et un groupement ammonium quaternaire. Il ne s'agit pas de bases d'oxydation.

Il a déjà été proposé, notamment dans le brevet US 5,139,532, d'utiliser certains dérivés cationiques de paraphénylènediamines, à savoir plus précisément des paraphénylènediamines dont un des groupements amino est monosubstitué par une chaîne aliphatique quaternisée, pour la teinture d'oxydation des fibres kératiniques dans des nuances intenses et plus rouges que celles obtenues habituellement en mettant en oeuvre des paraphénylènediamines classiques, c'est à dire ne portant pas de groupement cationique. Toutefois, l'utilisation des paraphénylènediamines décrites dans ce brevet antérieur ne permet pas d'obtenir une riche palette de couleurs et, de plus, les colorations obtenues ne donnent pas toujours entière satisfaction du point de vue de leur résistance vis à vis des diverses agressions que peuvent subir les cheveux (action de la lumière, de la transpiration, des shampooings, etc...).

Or, la demanderesse vient maintenant de découvrir, de façon totalement inattendue et surprenante, que certaines bases d'oxydation monobenzéniques de formule (I) définie ci-après, non seulement conviennent pour une utilisation comme précurseurs de colorant d'oxydation, mais en outre qu'elles permettent d'obtenir des compositions tinctoriales conduisant à des colorations puissantes couvrant une large palette de couleurs et présentant d'excellentes propriétés de résistances aux différents traitements que peuvent subir les fibres kératiniques. Enfin, ces composés s'avèrent être aisément synthétisables.

Ces découvertes sont à la base de la présente invention.

L'invention a donc pour premier objet des composés de formule (I) suivante, et leurs sels d'addition avec un acide : 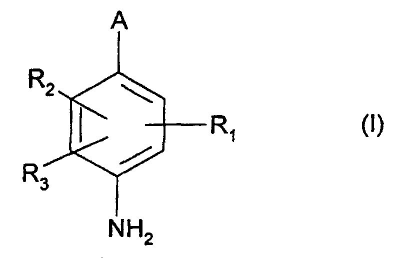 dans laquelle :
- R₁, R₂, R₃, qui peuvent être identiques ou différents, représentent un atome d'hydrogène ; un atome d'halogène ; un radical alkyl(C₁-C₆) carbonyle ; un radical aminoalkyl(C₁-C₆)carbonyle : un radical ; un radical N-alkyl(C₁-C₆)aminoalkyl(C₁-C₆)carbonyle ; un radical N,N-dialkyl(C₁-C₆)aminoalkyl(C₁ -C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonyl-alkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl-(C₁-C₆)carbamyle ; un radical carbamylalkyle(C₁-C₆) ; un radical N-alkyl(C₁-C₆)carbamylalkyle(C₁-C₆); un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-alkyl(C₁-C₆)aminoalkyl(C₁-C₆)carbonyle, N,N-dlalkyl(C₁-C₆)aminoalkyl(C₁-C₆)carbonyle, alkyl(C₁-C₆)carboxy, carbamyle, N-alkyl(C₁-C₆)carbamyle, N,N-dialkyl(C₁-C₆)carbamyle, alkyl(C₁-C₆)sulfonyle, aminosulfonyle, N-alkyl(C₁-C₆)aminosulfonyle, N,N-dialkyl(C₁-C₆)aminosulfonyle, thiocarbamyle, formyle ; ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle,
- R₆ désigne un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle ;
- A représente un groupement -NR₄R₅ ;
- R₄ et R₅, identiques, représentent un groupement Z ;
- Z représente un groupement de formule (II) suivante: 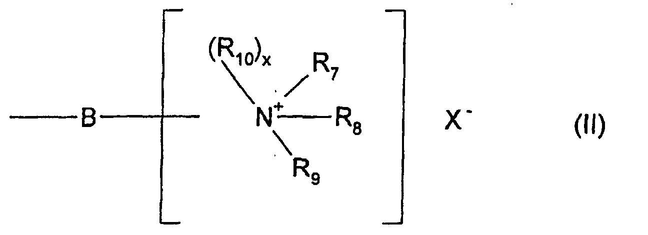 dans laquelle:
   - B est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, pouvant être substituée par un ou plusieurs radicaux hydroxyle ou alcoxy en C₁-C₆, et pouvant porter une ou plusieurs fonctions cétone ;
   - R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical carbamylalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
   - X ⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
   - R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
   - x est un nombre entier égal à 0 ou 1 ; avec les conditions suivantes :
      - lorsque x = 0, alors le bras de liaison B est rattaché à l'atome d'azote portant les radicaux R₇ à R₉ ;
      - lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison B est porté par un atome de carbone dudit cycle saturé ;
      étant entendu :

   - que lorsque A représente un groupement -NR₄R₅ dans lequel R₄ ou R₅ représente un groupement Z dans lequel le bras de liaison B représente une chaîne alkyle comportant une fonction cétone, alors ladite fonction cétone n'est pas directement rattachée à l'atome d'azote du groupement -NR₄R₅.

Comme indiqué précédemment, les colorations obtenues avec la composition de teinture d'oxydation conforme à l'invention sont puissantes et couvrent une large palette de couleurs. Elles présentent de plus d'excellentes propriétés de résistance vis à vis de l'action des différents agents extérieurs (lumière, intempéries, lavage, ondulation permanente, transpiration, frottements).

Dans la formule (I) ci-dessus les radicaux alkyle et alcoxy peuvent être linéaires ou ramifiés.

Parmi les composés de formule (I) ci-dessus, on peut notamment citer :
- le dichlorure de N,N-bis-(triméthylammonium-propyl)-4-amino-aniline ;
et ses sels d'addition avec un acide.

Les composés de formule (I) conformes à l'invention peuvent être facilement obtenus, selon des méthodes bien connues de l'état de la technique :
- soit par réduction des composés nitrés cationiques correspondants (para-nitranilines cationiques ou para-nitrophénols cationiques),
- soit par réduction des composés nitrosés cationiques correspondants (obtenus par exemple par nitrosation d'une aniline tertiaire ou d'un phénol correspondant),
- soit par réduction des composés azoïques cationiques correspondants (coupure réductrice).

Cette étape de réduction (obtention d'une amine aromatique primaire) qui confère au composé synthétisé son caractère de composé oxydable (de base d'oxydation) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quaternisation) et terminer par la "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Lorsque la synthèse est terminée, les composés de formule (I) conformes à l'invention peuvent, le cas échéant, être récupérés par des méthodes bien connues de l'état de la technique telles que la cristallisation ou la distillation.

Un autre objet de l'invention est l'utilisation des composés de formules (1) conformes à l'invention à titre de base d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (1) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) : 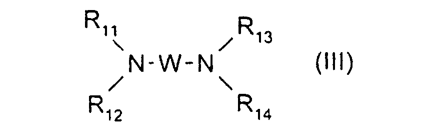 dans laquelle W est un reste propylène éventuellement substitué par un groupement hydroxyle ou un radical alkyle en C₁-C₆ ; R₁₁, R₁₂, R₁₃ et R₁₄, identiques ou différents, représentent un atome d'hydrogène, un radical alkyle en C₁-C₆ ou hydroxyalkyle en C₁-C₆.
La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines différentes des composés de formule (I) conformes à l'invention, les bis-phénylalkylènediamines, les para-aminophénols différents des composés de formule (I) conformes à l'invention, les ortho-aminophénols et les bases hétérocycliques.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylénediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.
Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyethyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 6-hydroxy benzomorpholine, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES DE PREPARATION

### EXEMPLE DE REFERENCE 1 : Synthèse du monochlorure, dichlorhydrate, monohydrate de [2-(2,5-diamino-phénoxy)-éthyl]-diéthylméthyl-ammonium

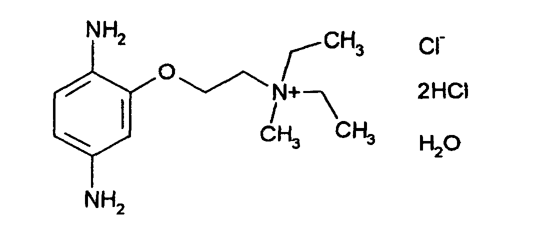

### a) Préparation du méthylsulfate de [2-(2-acétylamino-5-nitro-phénoxy)-éthyl]-diéthyl-méthyl-ammonium

La quaternisation de 59,1g (0,2 mole) de N-[2-(2-diéthylamino-éthoxy)-4-nitrophényl]-acétamide dissous dans 700 ml d'acétate d'éthyle a été faite en ajoutant 20,9 ml (0,22 mole) de diméthylsulfate sous agitation, pendant une heure, à température ambiante.
Le composé quaternisé a précipité.
On a ensuite chauffé le mélange réactionnel pendant une heure à 50°C.
On a essoré les cristaux, réempaté dans le minimum d'éthanol absolu et séché à 50°C sous vide et sur anhydride phosphorique.
On a obtenu 71,3g de cristaux jaune pâle qui fondaient à 141-145°C (Kofler) et dont l'analyse élémentaire calculée pour C₁₆H₂₇N₃O₈S était:

| % | C | H | N | O | S |
|---|---|---|---|---|---|
| Calculé | 45,60 | 6,46 | 9,97 | 30,37 | 7,61 |
| Trouvé | 45,18 | 6,44 | 9,84 | 30,93 | 7,49 |

### b) Réduction du méthylsulfate de [2-(2-acétylamino-5-nitro-phénoxy)éthyl]-diéthyl-méthyl-ammonium

On a chauffé au reflux de l'alcool un mélange de 100 ml d'éthanol à 96°, de 10 ml d'eau, de 50 g de zinc en poudre fine et de 1 g de chlorure d'ammonium.
On a ajouté par portions de façon à maintenir le reflux sans chauffage 42,1g (0,1 mole) de méthylsulfate de [2-(2-acétylamino-5-nitro-phénoxy)-éthyl]-diéthyl-méthyl-ammonium préparé à l'étape précédente. La réaction a été exothermique.
A la fin de l'addition on a maintenu le reflux pendant 10 minutes supplémentaires.
On a filtré bouillant et évaporé à sec sous pression réduite.
On a obtenu 44,4 g d'une huile jaune pâle de méthylsulfate de [2-(2-acétylamino-5-amino-phénoxy)-éthyl]-diéthyl-méthyl-ammonium.

### c) Désacétylation du méthylsulfate de [2-(2-acétylamino-5-amino-phénoxy)-éthyl]-diéthyl-méthyl-ammonium

Le méthylsulfate de [2-(2-acétylamino-5-amino-phénoxy)-éthyl]-diéthyl-méthylammonium, obtenu à l'étape précédente (43,9 g), a été mis en solution, à température ambiante et sous agitation, dans 100 ml d'éthanol absolu chlorhydrique environ 5N.
Au bout d'une demi-heure un précipité cristallisé blanc est apparu.
La suspension a été chauffée une heure au reflux de l'alcool pour compléter l'échange d'anions.
On a refroidi, essoré, lavé à l'éthanol absolu et séché à 50°C sous vide et sur potasse.
On a obtenu 28,9g de cristaux blancs qui ont fondu avec décomposition à 110-120°C (Kofler) et dont l'analyse élémentaire calculée pour C₁₃H₂₆N₃OCl₃ + H₂O était :

| % | C | H | N | O | Cl |
|---|---|---|---|---|---|
| Calculé | 42,81 | 7,74 | 11,52 | 8,77 | 29,16 |
| Trouvé | 43,18 | 7,59 | 11,30 | 8,43 | 29,80 |

### EXEMPLE DE PREPARATION 2 : Synthèse du dichlorhydrate, dichlorure de N,N-bis-(triméthyiammonium-propyl)-4-amino-aniline

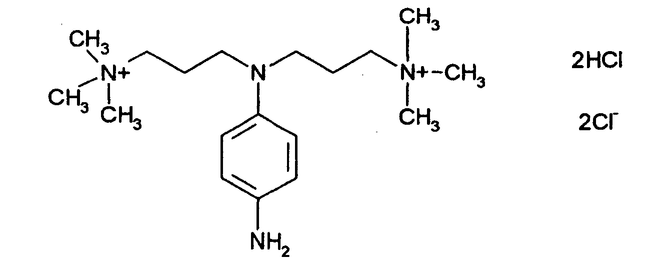

### a) Préparation de la N-(3-diméthylamino-propyl)-N',N'-diméthyl-N-(4-nitrophényl)-propane-1,3-diamine

Sous agitation on a chauffé pendant 2 heures au bain-marie bouillant un mélange de 28,2 g (0,2 mole) de 1-Fluoro-4-nitro-benzène, de 39,4 g (0,21 mole) de N-(3-diméthylamino-propyl)-N',N'-diméthyl-propane-1,3-diamine et de 27,6 g (0,2 mole) de carbonate de potassium dans 200 ml de diméthylsulfoxyde.
On a refroidi, dilué avec 400 ml d'eau et extrait à l'acétate d'éthyle.
On a lavé plusieurs fois à l'eau, séché sur sulfate de sodium, filtré et évaporé à sec sous pression réduite.
On a obtenu 61,6 g d'huile jaune de N-(3-diméthylamino-propyl)-N',N'-diméthyl-N-(4-nitro-phényl)-propane-1,3-diamine attendu.

### b) Préparation du di-méthylsulfate de N,N-bis-(triméthylammonium-propyl)-4-nitro-aniline

On a utilisé le mode opératoire décrit pour l'exemple 1, étape a) ci-dessus. A partir de 61,6 g (0,2 mole) de N-(3-diméthylamino-propyl)-N',N'-diméthyl-N-(4-nitro-phényl)-propane-1,3-diamine obtenu à l'étape précédente et de 42,1 ml (0,44 mole) de sulfate de méthyle, on a obtenu 88,5 g de cristaux jaunes de di-méthylsulfate de N,N-bis-(triméthylammonium-propyl)-4-nitro-aniline qui ont fondu à 196°C (Kofler) et dont l'analyse élémentaire calculée pour C₂₀H₄₀N₄O₁₀S₂ était :

| % | C | H | N | O | S |
|---|---|---|---|---|---|
| Calculé: | 42,84 | 7,19 | 9,99 | 28,54 | 11,44 |
| Trouvé: | 42,68 | 7,20 | 9,87 | 28,60 | 11,44 |

### c) Réduction du di-méthylsulfate de N,N-bis-(triméthylammonium-propyl)-4-nitro-aniline

Dans un hydrogénateur on a placé 78,5 g (0,14 mole) de di-méthylsulfate de N,N-bis-(triméthylammonium-propyl)-4-nitro-aniline, 15 g de palladium à 5% sur charbon (contenant 50% d'eau), 250 ml d'isopropanol et 250 ml d'eau.
La réduction s'est faite en une demi-heure sous une pression d'hydrogène d'environ 8 bars et à une température qui a été portée progressivement à 70°C. Après filtration du catalyseur sous azote on a coulé sur de l'acide chlorhydrique aqueux.
On a évaporé le filtrat à sec sous pression réduite et chauffé dans l'éthanol absolu chlorhydrique environ 5N pour compléter l'échange d'anions.
Après séchage à 40°C sous vide et sur potasse on a obtenu 51,4g de cristaux blancs qui ont fondu à 253-260°C (Kofler) et dont l'analyse élémentaire calculée pour C₁₈H₃₈N₄Cl₄ + ½ H₂O était :

| % | C | H | N | O | Cl |
|---|---|---|---|---|---|
| Calculé | 46,86 | 8,52 | 12,14 | 1,73 | 30,74 |
| Trouvé | 46,70 | 8,42 | 11,78 | 2,30 | 30,83 |

## Revendications

1. Composés de formule (I) suivante, et leurs sels d'addition avec un acide: 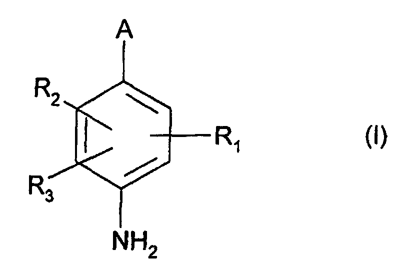 dans laquelle:
• R₁, R₂, R₃, qui peuvent être identiques ou différents, représentent un atome d'hydrogène ; un atome d'halogène ; un radical alkyl(C₁-C₆) carbonyle ; un radical aminoalkyl(C₁-C₆)carbonyle ; un radical ; un radical N-alkyl(C₁-C₆)aminoalkyl(C₁-C₆)carbonyle ; un radical N,N-dialkyl(C₁-C₆)aminoalkyl(C₁-C₆)carbonyle; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆); un radical carboxy ; un radical alkyl(C₁-C₆) carboxy; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle; un radical aminosulfonylalkyle(C₁-C₆) ; un radical N-alkyl(C₁-C₆)aminosulfonyl-alkyle(C₁-C₆); un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl-(C₁-C₆)carbamyle ; un radical carbamylalkyle(C₁-C₆); un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆)carboxy, carbamyle, N-alkyl(C₁-C₆)carbamyle, N,N-dialkyl(C₁-C₆)carbamyle, alkyl(C₁-C₆)sulfonyle, aminosulfonyle, , N-alkyl(C₁-C₆)aminosulfonyle, N,N-dialkyl(C₁-C₆)aminosulfonyle, thiocarbamyle, formyle, ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle,
• R₆ désigne un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle;
• A représente un groupement -NR₄R₅ ;
• R₄ et R₅, identiques, représentent un groupement Z ;
• Z représente un groupement de formule (II) suivante : 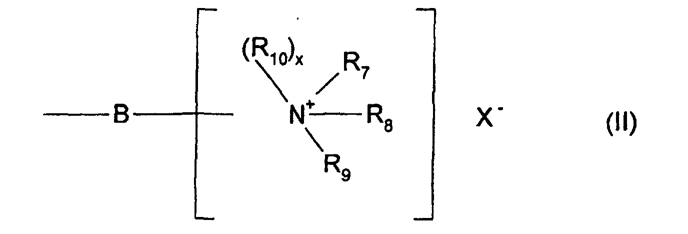 dans laquelle :
• B est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, pouvant être substituée par un ou plusieurs radicaux hydroxyle ou alcoxy en C₁-C₆, et pouvant porter une ou plusieurs fonctions cétone ;
• R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical carbamylalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
• X⁻ représente un anion monovalent ou divalent ;
• R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆
• x est un nombre entier égal à 0 ou 1 ; avec les conditions suivantes :
- lorsque x = 0, alors le bras de liaison B est rattaché à l'atome d'azote portant les radicaux R₇ à R₉ ;
- lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison B est porté par un atome de carbone dudit cycle saturé ;
étant entendu :
- que lorsque A représente un groupement -NR₄R₅ dans lequel R₄ ou R₅ représente un groupement Z dans lequel le bras de liaison B représente une chaîne alkyle comportant une fonction cétone, alors ladite fonction cétone n'est pas directement rattachée à l'atome d'azote du groupement -NR₄R₅ ;

2. Composés selon la revendication 1, **caractérisés par le fait que** X ⁻ représente un atome d'halogène tel que le chlore, le brome, le fluor ou l'iode, un hydroxyde, un hydrogènesulfate, ou un alkyl(C₁-C₆)sulfate.

3. Composé selon la revendication 1 ou 2, **caractérisé par le fait qu'**il est :
- le dichlorure de N,N-bis-(triméthylammonium-propyl)-4-amino-aniline ;
ou l'un de sels d'addition avec un acide.

4. Utilisation à titre de base d'oxydation pour la teinture d'oxydation des fibres kératiniques, et en particulier des fibres kératiniques humaines telles que les cheveux des composés de formule (I) ou de leurs sels d'addition avec un acide tels que définis dans l'une quelconque des revendications 1 à 3.

5. Composition pour la teinture d'oxydation des fibres kératiniques et en particulier des fibres kératiniques humaines telles que les cheveux, **caractérisée par le fait qu'**elle comprend, dans un milieu approprié pour la teinture, à titre de base d'oxydation, au moins un composé de formule (I) ou l'un de ses sels d'addition avec un acide tels que définis dans l'une quelconque des revendications 1 à 4.

6. 7. Composition selon la revendication 5 6, **caractérisée par le fait que** le ou les composés de formule (I) représentent de 0,0005 à 12 % en poids du poids total de la composition tinctoriale.

7. Composition selon la revendication 6 7, **caractérisée par le fait que** le ou les composés de formule (1) représentent de 0,005 à 6 % en poids du poids total de la composition tinctoriale.

8. Composition selon l'une quelconque des revendications 5 à 7, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

9. Composition selon l'une quelconque des revendications 5 à 8, **caractérisée par le fait qu'**elle présente un pH compris entre 3 et 12.

10. Composition selon l'une quelconque des revendications 5 à 9, **caractérisée par le fait qu'**elle renferme au moins une base d'oxydation additionnelle choisie parmi les paraphénylènediamines différentes des composés de formule (I), les bis-phénylalkylènediamines, les para-aminophénols différents des composés de formule (I), les ortho-aminophénols et les bases hétérocycliques.

11. Composition selon la revendication 10, **caractérisée par le fait que** la ou les bases d'oxydation additionnelles représentent de 0,0005 à 12 % en poids du poids total de la composition tinctoriale.

12. Composition selon l'une quelconque des revendications 5 à 10, **caractérisée par le fait qu'**elle renferme au moins un coupleur et/ou au moins un colorant direct.

13. Composition selon la revendication 12, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

14. Composition selon la revendication 12 ou 13, **caractérisée par le fait que** le ou les coupleurs représentent de 0,0001 à 10 % en poids du poids total de la composition tinctoriale.

15. Composition selon l'une quelconque des revendications 5 à 14, **caractérisée par le fait que** les sels d'addition avec un acide sont choisis parmi les chlorhydrates, les bromhydrates, les sulfates, les citrates, les succinates, les tartrates, les lactates et les acétates.

16. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 5 à 15, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 16, **caractérisé par le fait que** l'agent oxydant est choisi parmi le peroxyde d'hydrogène, le peroxyde d'urée, les bromates de métaux alcalins, les persels tels que les perborates et persulfates.

18. Dispositif à plusieurs compartiments, ou "kit" de teinture à plusieurs compartiments, dont un premier compartiment renferme une composition tinctoriale telle que définie à l'une quelconque des revendications 5 à 15 et un second compartiment renferme une composition oxydante.

## Patentansprüche

1. Verbindungen der folgenden Formel (I) und ihre Additionssalze mit einer Säure: 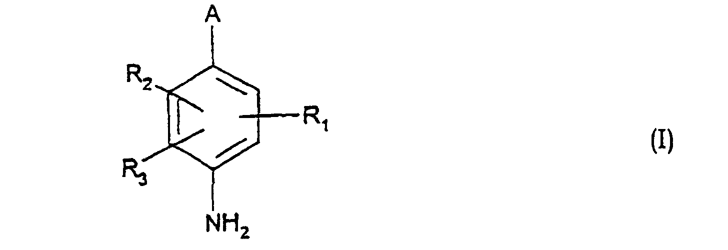 worin:
· R₁, R₂ und R₃, die identisch oder voneinander verschieden sein können, bedeuten Wasserstoff; Halogen; Alkyl(C₁₋₆)carbonyl; Aminoalkyl(C₁₋₆)carbonyl; N-Alkyl(C₁₋₆)aminoalkyl(C₁₋₆)carbonyl; N,N-Dialkyl(C₁₋₆)aminoalkyl(C₁₋₆)carbonyl; Aminoalkyl(C₁₋₆)carbonylalkyl(C₁₋₆); N-Alkyl(C₁₋₆)aminoalkyl(C₁₋₆)carbonylalkyl(C₁₋₆); N,N-Dialkyl(C₁₋₆)ammoalkyl(C₁₋₆)carbonylalkyl(C₁₋₆); Carboxy; Alkyl(C₁₋₆)-carboxy; Alkyl(C₁₋₆)sulfonyl; Aminosulfonyl; N-Alkyl(C₁₋₆)aminosulfonyl; N,N-Dialkyl(C₁₋₆)aminosulfonyl; Aminosulfonylalkyl(C₁₋₆); N-Alkyl(C₁₋₆)amino sulfonylalkyl(C₁₋₆); N,N-Dialkyl(C₁₋₆)amino-sulfonylalkyl(C₁₋₆), Carbamoyl, N-Alkyl(C₁₋₆) carbamoyl, N,N-Dialkyl(C₁₋₆)carbamoyl; Carbamoylalkyl(C₁₋₆); N-Alkyl(C₁₋₆)-carbamoylalkyl(C₁₋₆); N,N-Dialkyl(C₁₋₆)-carbamoylalkyl(C₁₋₆); C₁₋₆-Alkyl; C₁₋₆-Monohydroxyalkyl; C₂₋₆-Polyhydroxyalkyl; Alkoxy(C₁₋₆)-alkyl(C₁₋₆); C₁₋₆-Trifluoralkyl; Cyano; OR₆ oder SR₆; eine Aminogruppe, die mit Alkyl(C₁₋₆)carbonyl, Alkyl(C₁₋₆)carboxy, Trifluoralkyl(C₁₋₆)-carbonyl, Aminoalkyl(C₁₋₆)carbonyl, N-Alkyl(C₁₋₆)-aminoalkyl(C₁₋₆)carbonyl, N,N-Dialkyl(C₁₋₆)-aminoalkyl(C₁₋₆)carbonyl, Alkyl(C₁₋₆)carboxy, Carbamoyl, N-Alkyl(C₁₋₆)-carbamoyl, N,N-Dialkyl(C₁₋₆)carbamoyl, Alkyl(C₁₋₆)sulfonyl, Aminosulfonyl, N-Alkyl(C₁₋₆)-aminosulfonyl, N,N-Dialkyl(C₁₋₆)-aminosulfonyl, Thiocarbamoyl oder Formyl geschützt ist; oder C₁₋₆-Aminoalkyl, wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Carbamoyl, N-Alkyl(C₁₋₆)-carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy oder Thiocarbamoyl;
· die Gruppe R₆ bedeutet C₁₋₆-Alkyl; C₁₋₆-Monohydroxyalkyl; C₂₋₆-Pölyhydroxyalkyl; Alkoxy(C₁₋₆)alkyl(C₁₋₆); Aryl; Benzyl; C₁₋₆-Carboxyalkyl; Alkyl(C₁₋₆)carboxyalkyl(C₁₋₆); C₁₋₆-Cyanoalkyl; C₁₋₆-Carbamoylalkyl; N-Alkyl(C₁₋₆)carbamoylalkyl(C₁₋₆); N,N-Dialkyl(C₁₋₆)-carbamoylalkyl(C₁₋₆); C₁₋₆-Trifluoralkyl; C₁₋₆-Aminosulfonylalkyl; N-Alkyl(C₁₋₆) aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); C₁₋₆-Aminoalkyl; C₁₋₆-Aminoalkyl, wobei die Aminogruppe mit einer oder zwei Gruppen substituiert ist, die identisch oder voneinander verschieden sind und unter den folgenden Gruppen ausgewählt sind: C₁₋₆-Alkyl, C₁₋₆ Monohydroxyalkyl, C₂₋₆-Polyhydroxyalkyl, Alkyl(C₁₋₆)carbonyl, Formyl, Trifluoralkyl(C₁₋₆)carbonyl, Alkyl(C₁₋₆)carboxy, Carbamoyl, N-Alkyl(C₁₋₆)carbamoyl, N,N-Dialkyl(C₁₋₆)carbamoyl, Thiocarbamoyl oder Alkyl(C₁₋₆)sulfonyl;
· die Gruppe A bedeutet eine Gruppe -NR₄R₅;
· die Gruppen R₄ und R₅, die identisch sind, bedeuten eine Gruppe Z;
· Z ist eine Gruppe der folgenden Formel (II): 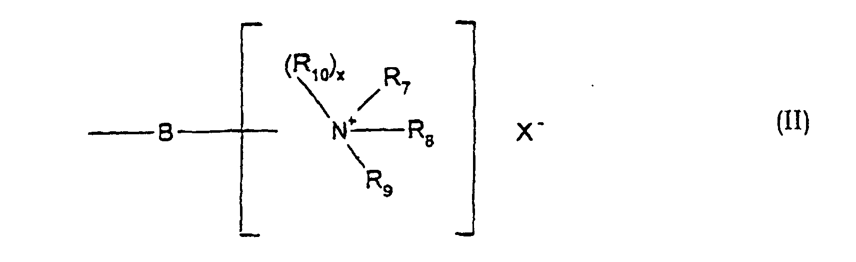
worin bedeuten:
· B eine Verbindungsgruppe, die eine Alkylkette mit vorzugsweise 1 bis 14 Kohlenstoffatome bedeutet, die geradkettig oder verzweigt vorliegt und mit einem oder mehreren Heteroatomen, wie beispielsweise Sauerstoff, Schwefel oder Stickstoff, unterbrochen sein kann, mit einer oder mehreren Hydroxygruppen oder C₁₋₆-Alkoxygruppen substituiert sein kann und eine oder mehrere Ketogruppen tragen kann;
· R₇, R₈ oder R₉, die gleich oder verschieden sind, C₁₋₆-Alkyl, C₁₋₆-Monohydroxyalkyl, C₂₋₆-Polyhydroxyalkyl, Alkoxy(C₁₋₆) alkyl(C₁₋₆), C₁₋₆-Cyanoalkyl, Aryl, Benzyl, C₁₋₆-Carbamoylalkyl, Trialkyl(C₁₋₆)silanalkyl(C₁₋₆) oder C₁₋₆ Aminoalkyl, wobei die Aminogruppe mit Alkyl(C₁₋₆)carbonyl, Carbamoyl oder Alkyl(C₁₋₆)sulfonyl geschützt ist; zwei der Gruppen R₇, R₈ und R₉ können auch gemeinsam mit dem Stickstoffatom, an das sie gebunden sind, einen 5- oder 6-gliedrigen Ring bilden, der Kohlenstoffatome enthält oder ein oder mehrere Heteroatome aufweist, wobei der Ring unsubstituiert vorliegen kann oder substituiert sein kann mit Halogen, Hydroxy, C₁₋₆-Alkyl, C₁₋₆-Monohydroxyalkyl, C₂₋₆-Polyhydroxyalkyl, Nitro, Cyano, C₁₋₆-Cyanoalkyl, C₁₋₆-Alkoxy, Trialkyl(C₁₋₆)silanalkyl(C₁₋₆), Amido, Aldehydo, Carboxy, C₁₋₆-Alkylcarbonyl, Thio, C₁₋₆-Thioalkyl, Alkyl(C₁₋₆)thio, Amino, einer Aminogruppe, die mit Alkyl(C₁₋₆)carbonyl, Carbamoyl oder Alkyl(C₁₋₆)sulfonyl geschützt ist;
· die Gruppe X⁻ ein einwertiges oder zweiwertiges Anion;
· die Gruppe R₁₀ C₁₋₆-Alkyl; C₁₋₆-Monohydroxyalkyl; C₂₋₆-Polyhydroxyalkyl; Aryl; Benzyl; C₁₋₆-Aminoalkyl; C₁₋₆-Aminoalkyl, wobei die Aminogruppe mit Alkyl(C₁₋₆)carbonyl, Carbamoyl oder Alkyl(C₁₋₆)sulfonyl geschützt ist; C₁₋₆-Carboxyalkyl; C₁₋₆-Cyanoalkyl; C₁₋₆-Carbamoylalkyl; C₁₋₆-Trifluoralkyl; Trialkyl(C₁₋₆)silanalkyl(C₁₋₆); C₁₋₆-Sulfonamidoalkyl; Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)carbamoylalkyl(C₁₋₆); N-Alkyl(C₁₋₆)sulfonamidoalkyl(C₁₋₆);
· x 0 oder 1; mit den folgenden Bedingungen:
- die Verbindungsgruppe B ist an das Stickstoffatom gebunden, das die Gruppen R₇ bis R₉ trägt, wenn x = 0,
- zwei der Gruppen R₇ bis R₉ bilden gemeinsam mit dem Stickstoffatom, an das sie gebunden sind, einen oben definierten gesättigten 5- oder 6-gliedrigen Ring und die Verbindungsgruppe B wird von einem Kohlenstoffatom des gesätgten Rings getragen, wenn x = 1;
mit der Maßgabe, dass:
- wenn A eine Gruppe -NR₄R₅ ist, worin R₄ und R₅ eine Gruppe Z bedeuten, in der die Verbindungsgruppe B eine Alkylgruppe mit Ketogruppe ist, ist die Ketogruppe nicht direkt an das Stickstoffatom der Gruppe -NR₄R₅ gebunden.

2. Verbindungen nach Anspruch 1, **dadurch gekennzeichnet, dass** X⁻ ein Halogenatom, wie Chlor, Brom, Fluor oder Iod, Hydroxid, Hydrogensulfat oder Alkyl(C₁₋₆)sulfat bedeutet.

3. Verbindungen nach Anspruch 1 oder 2, **dadurch gekennzeichnet, dass** es sich um das N,N-Bis(trimethylammoniumpropyl)-4-aminoanilin-dichlorid oder ein Additionssalz dieser Verbindung mit einer Säure handelt.

4. Verwendung von Verbindungen der Formel (I) oder ihren Additionssalzen mit einer Säure nach einem der Ansprüche 1 bis 3 als Oxidationsbasen zum oxidativen Färben von Keratinfasern und insbesondere menschlichen Keratinfasem, wie dem Haar.

5. Zusammensetzung zum oxidativen Färben von Keratinfasern und insbesondere menschlichen Keratinfasern, wie dem Haar, **dadurch gekennzeichnet, dass** sie in einem zum Färben geeigneten Medium als Oxidationsbase mindestens eine Verbindung der Formel (I) oder eines ihrer Additionssalze mit einer Säure nach einem der Ansprüche 1 bis 4 enthält.

6. Zusammensetzung nach Anspruch 5, **dadurch gekennzeichnet, dass** die Verbindung(en) der Formel (I) 0,0005 bis 12 Gew.-% des Gesamtgewichts der Farbmittelzusammensetzung ausmachen.

7. Zusammensetzung nach Anspruch 6, **dadurch gekennzeichnet, dass** die Verbindung(en) der Formel (I) 0,005 bis 6 Gew.-% des Gesamtgewichts der Farbmittelzusammensetzung ausmachen.

8. Zusammensetzung nach einem der Ansprüche 5 bis 7, **dadurch gekennzeichnet, dass** das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glykolen und Glykolethern, aromatischen Alkoholen, analogen Produkten und deren Gemischen ausgewählt ist.

9. Zusammensetzung nach einem der Ansprüche 5 bis 8, **dadurch gekennzeichnet, dass** sie einen pH-Wert im Bereich von 3 bis 12 aufweist.

10. Zusammensetzung nach einem der Ansprüche 5 bis 9, **dadurch gekennzeichnet, dass** sie mindestens eine zusätzliche Oxidationsbase enthält, die unter den p-Phenylendiaminen, die von den Verbindungen der Formel (I) verschieden sind, den Bisphenylalkylendiaminen, den p-Aminophenolen, die von den Verbindungen der Formel (I) verschieden sind, den o-Aminophenolen und den heterocyclischen Basenausgewählt ist.

11. Zusammensetzung nach Anspruch 10, **dadurch gekennzeichnet, dass** die zusätzliche(n) Oxidationsbase(n) 0,0005 bis 12 Gew.-% des Gesamtgewichts der Farbmittelzusammensetzung ausmachen.

12. Zusammensetzung nach einem der Ansprüche 5 bis 10, **dadurch gekennzeichnet, dass** sie mindestens einen Kuppler und/oder mindestens einen Direktfarbstoff enthält.

13. Zusammensetzung nach Anspruch 12, **dadurch gekennzeichnet, dass** der oder die Kuppler unter den m-Phenylendiaminen, m-Aminophenolen, m-Dihydroxybenzolen und den heterocyclischen Kupplern und deren Additionssalzen mit einer Säure ausgewählt sind.

14. Zusammensetzung nach Anspruch 12 oder 13, **dadurch gekennzeichnet, dass** der oder die Kuppler 0,0001 bis 10 Gew.-% des Gesamtgewichts der Farbmittelzusammensetzung ausmachen.

15. Zusammensetzung nach einem der Ansprüche 5 bis 14, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

16. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 5 bis 15 aufgebracht wird, wobei die Farbe bei einem säuren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

17. Verfahren nach Anspruch 16, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, ausgewählt ist.

18. Vorrichtung mit mehreren Abteilungen oder "Kit" zum Färben mit mehreren Abteilungen, wobei eine erste Abteilung eine Farbmittelzusammensetzung nach einem der Ansprüche 5 bis 15 und eine zweite Abteilung eine oxidierende Zusammensetzung enthält.

## Claims

1. Compounds of formula (I) below, and the addition salts thereof with an acid: 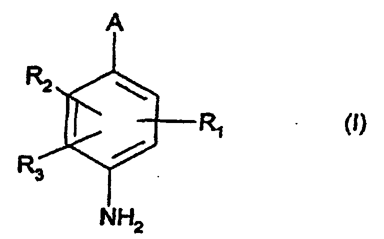 in which:
• R₁, R₂ and R₃, which may be identical or different, represent a hydrogen atom; a halogen atom; a (C₁-C₆)alkylcarbonyl radical; an amino(C₁-C₆)alkylcarbonyl radical; an N-(C₁-C₆) alkylamino(C₁-C₆)-alkylcarbonyl radical; an N,N-di (C₁-C₆)alkylamino(C₁-C₆)alkylcarbonyl radical; an amino(C₁-C₆)alkylcarbonyl (C₁-C₆) alkyl radical; an N-(C₁-C₆) alkylamino(C₁-C₆)alkylcarbonyl(C₁-C₆)alkyl radical; an N,N-di(C₁-C₆) alkylamino (C₁-C₆) alkylcarbonyl (C₁-C₆)alkyl radical; a carboxyl radical; a (C₁-C₆)alkylcarboxyl radical; a C₁-C₆ alkylsulphonyl radical; an aminosulphonyl radical; a C₁-C₆ N-alkylaminosulphonyl radical; an N,N-di(C₁-C₆)alkylaminosulphonyl radical; a C₁-C₆ aminosulphonylalkyl radical; an N-(C₁-C₆) alkylaminosulphonyl (C₁-C₆) alkyl radical; an N,N-di (C₁-C₆)alkylaminosulphonyl (C₁-C₆)alkyl radical; a carbamyl radical; an N-(C₁-C₆)-alkylcarbamyl radical; an N,N-di(C₁-C₆)alkylcarbamyl radical; a carbamyl(C₁-C₆)alkyl radical; an N-(C₁-C₆)alkylcarbamyl (C₁-C₆) alkyl radical; an N,N-di(C₁-C₆)alkylcarbamyl (C₁-C₆)alkyl radical; a C₁-C₆ alkyl radical; a C₁-C₆ monohydroxyalkyl radical; a C₂-C₆ polyhydroxyalkyl radical; a (C₁-C₆) alkoxy (C₁-C₆) alkyl radical; a C₁-C₆ trifluoroalkyl radical; a cyano radical; a group OR₆ or SR₆; an amino group protected with a (C₁-C₆)alkylcarbonyl, (C₁-C₆)alkylcarboxyl, trifluoro(C₁-C₆)alkylcarbonyl, amino (C₁-C₆) alkylcarbonyl, N-(C₁-C₆) alkylamino (C₁-C₆) alkylcarbonyl, N,N-di (C₁-C₆) alkylamino (C₁-C₆) alkylcarbonyl, (C₁-C₆)alkylcarboxyl, carbamyl, N-(C₁-C₆)alkylcarbamyl, N,N-di (C₁-C₆) alkylcarbamyl, C₁-C₆ alkylsulphonyl, aminosulphonyl, C₁-C₆ N-alkylaminosulphonyl, N,N-di (C₁-C₆)alkylaminosulphonyl, thiocarbamyl or formyl radical; or a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, carbamyl, N-(C₁-C₆)alkyl-carbamyl, N,N-di (C₁-C₆)alkylcarbamyl, C₁-C₆ alkylsulphonyl, formyl, trifluoro (C₁-C₆) alkylcarbonyl, (C₁-C₆)alkylcarboxyl and thiocarbamyl radicals;
• R₆ denotes a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; an aryl radical; a benzyl radical; a carboxy(C₁-C₆)-alkyl radical; a (C₁-C₆) alkylcarboxy (C₁-C₆) alkyl radical; a cyano(C₁-C₆)alkyl radical; a carbamyl(C₁-C₆)alkyl radical; an N-(C₁-C₆)alkylcarbamyl(C₁-C₆)alkyl radical; an N,N-di (C₁-C₆)alkylcarbamyl(C₁-C₆)alkyl radical; a C₁-C₆ trifluoroalkyl radical; a C₁-C₆ aminosulphonylalkyl radical; an N- (C₁-C₆) alkylaminosulphonyl (C₁-C₆) alkyl radical; an N,N-di (C₁-C₆) alkylaminosulphonyl (C₁-C₆)alkyl radical; a (C₁-C₆)alkylsulphinyl (C₁-C₆) alkyl radical; a (C₁-C₆) alkylsulphonyl (C₁-C₆) alkyl radical; a (C₁-C₆) alkylcarbonyl (C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆) alkylcarbonyl, formyl, trifluoro- (C₁-C₆) alkylcarbonyl, (C₁-C₆) alkylcarboxyl, carbamyl, N-(C₁-C₆)alkylcarbamyl, N,N-di(C₁-C₆)alkylcarbamyl, thiocarbamyl and C₁-C₆ alkylsulphonyl radicals;
• A represents a group -NR₄R₅;
• R₄ and R₅, which are identical, represent a group Z;
• Z represents a group of formula (II) below: 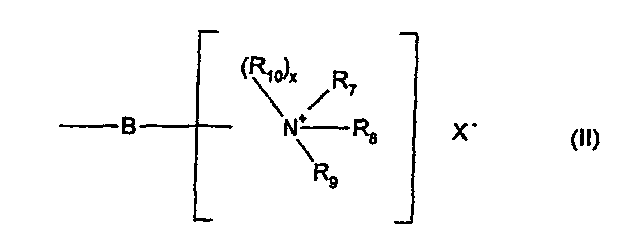 in which:
• B is a linker arm which represents a linear or branched alkyl chain preferably containing from 1 to 14 carbon atoms, which can be interrupted by one or more hetero atoms such as oxygen, sulphur or nitrogen atoms, and which can be substituted with one or more hydroxyl or C₁-C₆ alkoxy radicals, and which can bear one or more ketone functions;
• R₇, R₈ and R₉, which may be identical or different, represent a C₁-C₆ alkyl radical, a C₁-C₆ monohydroxyalkyl radical, a C₂-C₆ polyhydroxyalkyl radical, a (C₁-C₆)alkoxy(C₁-C₆)alkyl radical, a cyano(C₁-C₆)alkyl radical, an aryl radical, a benzyl radical, a C₁-C₆ carbamylalkyl radical, a tri(C₁-C₆)alkylsilane(C₁-C₆)alkyl radical or a C₁-C₆ aminoalkyl radical in which the amine is protected with a (C₁-C₆)alkylcarbonyl, carbamyl or C₁-C₆ alkylsulphonyl radical; two of the radicals R₇, R₈ and R₉ can together also form, with the nitrogen atom to which they are attached, a saturated 5- or 6-membered carbon ring or a ring containing one or more hetero atoms, it being possible for the said ring to be substituted or unsubstituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri (C₁-C₆) alkylsilane (C₁-C₆) alkyl radical, an amido radical, an aldehydo radical, a carboxyl radical, a (C₁-C₆)alkylcarbonyl radical, a thio radical, a C₁-C₆ thioalkyl radical, a C₁-C₆ alkylthio radical, an amino radical or an amino radical protected with a (C₁-C₆)alkylcarbonyl, carbamyl or C₁-C₆ alkylsulphonyl radical;
• X⁻ represents a monovalent or divalent anion;
• R₁₀ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆)alkylcarbonyl, carbamyl or C₁-C₆ alkylsulphonyl radical; a carboxy(C₁-C₆)alkyl radical; a cyano(C₁-C₆)-alkyl radical; a carbamyl(C₁-C₆)alkyl radical; a C₁-C₆ trifluoroalkyl radical; a tri(C₁-C₆)-alkylsilane(C₁-C₆)alkyl radical; a C₁-C₆ sulphonamidoalkyl radical; a (C₁-C₆)alkyl-carboxy(C₁-C₆)alkyl radical; a (C₁-C₆)alkyl-sulphinyl (C₁-C₆)alkyl radical; a (C₁-C₆)-alkylsulphonyl(C₁-C₆)alkyl radical; a (C₁-C₆) alkylketo (C₁-C₆)alkyl radical; an N-(C₁-C₆)alkylcarbamyl(C₁-C₆)alkyl radical; an N-(C₁-C₆)alkylsulphonamido(C₁-C₆)alkyl radical;
• x is an integer equal to 0 or 1; with the following conditions:
- when x = 0, then the linker arm B is attached to the nitrogen atom bearing the radicals R₇ to R₉;
- when x = 1, then two of the radicals R₇ to R₉ form, together with the nitrogen atom to which they are attached, a 5- or 6-membered saturated ring as defined above; and the linker arm B is borne by a carbon atom of the said saturated ring;
it being understood that:
- when A represents a group -NR₄R₅ in which R₄ or R₅ represents a group Z in which the linker arm B represents an alkyl chain comprising a ketone function, then the said ketone function is not directly attached to the nitrogen atom of the group -NR₄R₅.

2. Compounds according to Claim 1, **characterized in that** X⁻ represents a halogen atom such as chlorine, bromine, fluorine or iodine, a hydroxide, a hydrogen sulphate or a C₁-C₆ alkyl sulphate.

3. Compound according to Claim 1 or 2, **characterized in that** it is:
- N,N-bis(trimethylammoniumpropyl)-4-aminoaniline dichloride;
or one of the addition salts thereof with an acid.

4. Use, as oxidation bases for the oxidation dyeing of keratin fibres, and in particular of human keratin fibres such as the hair, of the compounds of formula (I) or of the addition salts thereof with an acid as defined in any one of Claims 1 to 3.

5. Composition for the oxidation dyeing of keratin fibres, and in particular of human keratin fibres such as the hair, **characterized in that** it comprises, in a medium that is suitable for dyeing, as oxidation bases, at least one compound of formula (I) or an addition salt thereof with an acid as defined in any one of Claims 1 to 4.

6. Composition according to Claim 5, **characterized in that** the compound(s) of formula (I) represent(s) from 0.0005% to 12% by weight relative to the total weight of the dye composition.

7. Composition according to Claim 6, **characterized in that** the compound(s) of formula (I) represent(s) from 0.005% to 6% by weight relative to the total weight of the dye composition.

8. Composition according to any one of Claims 5 to 7, **characterized in that** the medium that is suitable for dyeing (or support) consists of water or of a mixture of water and at least one organic solvent chosen from C₁-C₄ lower alkanols, glycerol, glycols and glycol ethers, aromatic alcohols, similar products and mixtures thereof.

9. Composition according to any one of Claims 5 to 8, **characterized in that** it has a pH of between 3 and 12.

10. Composition according to any one of Claims 5 to 9, **characterized in that** it contains at least one additional oxidation base chosen from para-phenylenediamines other than the compounds of formula (I), bis(phenyl)alkylenediamines, para-aminophenols other than the compounds of formula (I), ortho-aminophenols and heterocyclic bases.

11. Composition according to Claim 10, **characterized in that** the additional oxidation base(s) represent(s) from 0.0005% to 12% by weight relative to the total weight of the dye composition.

12. Composition according to any one of Claims 5 to 10, **characterized in that** it contains at least one coupler and/or at least one direct dye.

13. Composition according to Claim 12, **characterized in that** the coupler(s) is (are) chosen from meta-phenylenediamines, meta-aminophenols, metadiphenols and heterocyclic couplers, and the addition salts thereof with an acid.

14. Composition according to Claim 12 or 13, **characterized in that** the coupler(s) represent(s) from 0.0001% to 10% by weight relative to the total weight of the dye composition.

15. Composition according to any one of Claims 5 to 14, **characterized in that** the addition salts with an acid are chosen from the hydrochlorides, hydrobromides, sulphates, citrates, succinates, tartrates, lactates and acetates.

16. Process for dyeing keratin fibres, and in particular human keratin fibres such as the hair, **characterized in that** at least one dye composition as defined in any one of Claims 5 to 15 is applied to these fibres, and the colour is developed at acidic, neutral or alkaline pH using an oxidizing agent that is added to the dye composition just at the time of use or that is present in an oxidizing composition applied simultaneously or sequentially in a separate manner.

17. Process according to Claim 16, **characterized in that** the oxidizing agent is chosen from hydrogen peroxide, urea peroxide, alkali metal bromates and persalts such as perborates and persulphates.

18. Multi-compartment device or multi-compartment dyeing "kit", a first compartment of which contains a dye composition as defined in any one of Claims 5 to 15, and a second compartment of which contains an oxidizing composition.
